# EUROPEAN PATENT APPLICATION

(11) **EP 1 118 311 A2**
(43) Date of publication of application: **25.07.2001**
(21) Application number: 01100019.7
(22) Date of filing: 04.01.2001
(51) Int. Cl.: A61B 18/20

(54) **Apparatus for laser light irradiation to living body**

(30) Priority: 06.01.2000 JP 2000000604
(71) Applicant: S.L.T. Japan Co., Ltd., Hachioji-shi, Tokyo 192-0081 (JP)
(72) Inventor: Daikuzono, Norio, Cincinnati, Ohio 45242 (US)
(74) Representative: Held, Stephan, Dr.rer.nat., Dipl.-Chem.

(57) **Abstract**

The laser light irradiating apparatus of the present invention is adapted to irradiate the surface of the living body tissue, such as the blotch of the skin or the inside of the tissue with laser light from laser light supplying means.
The apparatus comprises laser light transmitting member having a rear end to which the laser light from said laser light supplying means is incident and a front end from which the laser light is emitted; holding means for holding said laser light transmitting member; turning means for moving the front end portion of the laser light transmitting member having at least its front end substantially along a turning locus around a turning center axis within said holding means; and a reflector cylinder for reflecting laser light on its inner surface which is mounted on said holding means coaxially with said turning center axis and is disposed in front of at least said front end of said laser light transmitting member for reflecting part of the laser light emitted from said front end of said laser light transmitting member on its inner surface so that the reflected light is incident upon said tissue.

## Description

### Field of the Invention

The present invention relates to an apparatus for irradiating a living body with laser light, and in particular to an apparatus for irradiating a living body with laser light which is suitable for removing the blotch, hair on the surface of the skin and the tartar of the teeth.

### Background of the Invention

Recently, removal of the blotch has been conducted by irradiating the surface of the tissue of the blotch with laser light for evaporating the tissue.

Since the laser light emitted from the tip end of an optical fiber exhibits a Gaussian distribution (normal distribution) in its energy, the energy of the laser light which is in the center of the irradiation is highest when the tissue is irradiated with laser light, so that the energy is abruptly lowered in an outer radial direction from the center to the periphery. Accordingly, it is necessary to preset such an energy of the laser light in the center of irradiation that the laser light will evaporate the blotch tissue without giving any damage to the normal tissue on exposure the laser light to the surface of the blotch tissue.

Since the laser light emitted from the tip end of the optical fiber exhibits the Gaussian distribution in its energy as mentioned above, the energy of the laser light which irradiates the tissue surface has a tendency that it is abruptly lowered as the irradiation position is shifted from the irradiation center. Accordingly, the area of the tissue which can be evaporated by the irradiation is very small.

As a result, a surgeon has to repeat the irradiation with the laser light many times by changing the position of the laser with respect to the surface of the blotch of a patient and it takes a long period of time to finish the operation. The area, the tissue of which can not be evaporated may often remain among the adjacent irradiation positions, resulting in an irregular treatment.

Thus, development of an apparatus which is capable of uniformly irradiating a relatively wide area with laser light has been demanded.

At this end, Japanese Unexamined Patent Publication No. Tokkai sho 58-83973 and Japanese Examined Patent Publication No. Tokko hei 8-111 disclose a technology which is capable of uniformly irradiating with laser light by using a "uniform light irradiating rod".

However, this prior art uses a combination of the "uniform light irradiating rod" with an optical system such as lens and it is expensive to manufacture it and there is the risk that the optical system may be contaminated with the evaporated components of the tissue, so that uniform irradiation with laser light becomes impossible. Furthermore, it is not suitable for uniformly irradiating a wide area with laser light.

Therefore, it is an object of the present invention to provide an apparatus which is capable of uniformly irradiating a wider area with laser light in a positive manner.

### Summary of the Invention

In accordance with the present invention, there is provided a laser light irradiating apparatus for irradiating the surface or inside of a living body tissue with laser light from laser light supplying means, comprising:
laser light transmitting member having a rear end to which the laser light from said laser light supplying means is incident and a front end from which the laser light is emitted;
holding means for holding said laser light transmitting member; and
turning means for moving the front end portion of the laser light transmitting member having at least its front end substantially along a turning locus around a turning center axis within said holding means.

In the present invention, the laser light from said light generating means is incident on the rear end of the laser light transmitting member and is emitted from the front end thereof. The laser light transmitting member is held by the holding member. While being held within the holding means, the laser light transmitting member is moved along the turning locus around the turning center axis by the turning means. It is preferable to turn the laser light transmitting member at, for example, r.p.m. of a few tens to ten thousands.

The laser light is emitted from the front end of the laser light transmitting member at each position in the course of the movement of the laser light transmitting member along said turning locus, that is in the course of high speed turning to exhibit Gaussian distribution. As a result, the energy distribution of the laser light in a cross-section which intersects the turning center axis depends upon the diameter of the turning locus S. It exhibits the triangular shape having a gradual summit when the diameter is small. It exhibits the shape of somma when the diameter is large. In either case, the energy distribution of the laser light of the present invention which irradiates the skin surface exhibits a uniform energy distribution having a low peak height and which is relatively higher over a wider area in contrast to the Gaussian distribution in the prior art in which the position of the laser transmitting member is always fixed, that is the energy distribution in which the peak in the center is higher and the gradient is steep and the slope is narrow.

Therefore, by appropriately selecting the relation among the diameter of the turning locus, distance between the tip end of the laser light transmitting member and the tissue, and the energy of the laser light which is emitted from the tip end of the laser light transmitting member, the entire area of the object of the tissue M can be irradiated with the laser light so that the energy distribution over the tissue M is uniform. As a result, the tissue can be positively and uniformly irradiated with the laser light over the wider area thereof without giving any local damage to the tissue.

In the present invention, it is preferable to dispose a reflector cylinder which reflects the laser light on the inner surface in front of the tip end of the laser light transmitting member in an coaxial manner with respect to the turning center axis so that the reflected laser light is incident upon the tissue. Part of the laser light emitted from the front end of the laser light transmitting member is reflected on the inner surface of the reflected cylinder and is incident upon the tissue. Accordingly, since the laser light which is reflected on the inner surface 20A of the reflector cylinder 20 is additively incident upon the tissue, the irradiation energy of the laser light on the tissue exhibits substantially inverted U shape, so that the wider area of the object tissue can be uniformly irradiated with laser light.

The reflector cylinder may be transparent or semi-transparent. In this configuration, the inner tissue is visible through the reflector cylinder. In case of evaporation of the skin blotch, irradiation of the laser light is enabled while confirming the position of the blotch during treatment. When the reflector cylinder is applied to the surface of the tissue, it may serve as a member for keeping the distance between the tissue and the front end of the laser light transmitting member.

The reflector cylinder may be removably mounted on the holding means. A plurality of kinds of reflector cylinders having different lengths are provided. By changing the manner of reflecting of the laser light on the inner surface 20A by selecting an appropriate reflecting cylinder among the plurality of reflector cylinders depending upon the situation, the irradiating energy distribution of the laser light with respect to the tissue can be changed. The energy of the laser light whichi s incident upon the tissue can also be changed even if the energy of the laser light emitted from the front end of the laser light transmitting member is made constant.

In an embodiment in which the reflector cylinder is mounted on the holding means coaxially with respect to the turning center axis, there may be provided cooling medium pumping means for pumping a cooling medium into the reflector cylinder to cool the surface of the tissue. Since the skin is irradiated with laser light without applying anesthesia, the skin which is irradiated with the laser light may be heated so that the patient can not endure it. In this case, the surface of the tissue can be cooled by pumping a cooling medium into the reflector cylinder for mitigating the hot feeling of the patient. If the reflector cylinder is formed on its wall with the opening, the cooling medium would formed on its wall with the opening, the cooling medium would be pumped out from an opening so that the heat which is recovered by the cooling medium can be discharged to the outside.

The cooling medium can be pumped into the inside of the reflector cylinder via the holding means.

The holding means may comprise a casing and a guide whichi s disposed within said casing in such a manner that it is rotatable around a turning center axis. The laser light transmitting member may be an optical fiber. The turning means may be a motor whichi s disposed within said holding means. At least front end portion of said optical fiber is held by being inserted into said guide with play at a position offset from said turning center axis in such a manner that it is in substantially parallel with said turning center axis. The guide is disposed to be rotated by a torque from said motor. Since the optical fiber is inserted into the guide with play in parallel with the turning center axis at this time, the optical fiber only traces a turning locus without following the rotation of the guide, that is, without being not twisted.

The torque of said motor may be transmitted to said guide via a gear mechanism for rotating the guide. Adopting a gear mechanism for transmitting the torque makes the apparatus more compact.

The laser light supplying means is capable of supplying pulsated laser light to said laser light transmitting member. Use of pulsated laser light allows the laser light having a high energy to be given to the tissue within a shorter interval.
Heat feeling for the patient is less in comparison with use of continuous wave.

In case in which pulsated laser light is used, it is preferable to design the apparatus so that the ratio of the number of teeth of the gear on the side of the guide of the gear mechanism to that of the gear on the side of the motor is not a whole number, but a fraction. If the pulsated laser light is irradiated at a given interval and the turning speed is constant, there is the possibility that the tissue is irradiated with the laser light at the same position on the turning locus. If the ratio of the number of the teeth of the gear on the side of the guide of the gear mechanism to that of the gear on the side of the motor is not a whole number, but a fraction, the tissue is irradiated with the laser light at different positions on the turning locus. Accordingly, the tissue can be uniformly irradiated with the laser light over an entire area within a predetermined period of time.

A plurality of laser light transmitting members may be provided. They may be provided at different positions on the turning locus, or alternatively may be held on the holding means at different radius positions from the turning center axis. If the plurality of laser transmitting members are provided, the irradiation energy which is a multiple times as much as the number of the laser light transmitting members can be supplied per unit time. The embodiment in which the laser light transmitting members are provided at different radial positions far from the turning center axis in effective to provide a wider irradiation area.

On treatment of the skin of the living body with lasers light irradiation, YAG pulsated laser light is excellent in treatment efficiency.

### Brief Description of the Drawings

Fig. 1 is a schematic view explaining the present invention]
Fig. 2 is a schematic view showing the manner of irradiating with laser light;
Fig. 3 is a longitudinal sectional view showing an exemplary laser light irradiating apparatus;
Fig. 4 is an enlarged longitudinal sectional view showing a front part of the apparatus shown in Fig. 3;
Fig. 5 is an enlarged longitudinal sectional view showing a rear part of the apparatus shown in Fig. 3;
Fig. 6 is a view explaining the manner in which the optical fiber traces an elliptical turning locus;
Fig. 7 is a view explaining an example in which the optical axis is inclined with respect to the turning center axis; and
Fig. 8 is a view explaining an example in which a plurality of optical fibers are provided.

### Mode of Embodying a Preferred Embodiment

Now, an embodiment of the present invention in which the skin blotch on the surface of the living tissue is removed will be described with reference to the drawings. The apparatus for irradiating a living body with laser light of the present invention is applicable to the removal of the hair and the tartar of the teeth as well as the removal of the blotch on the skin surface. For example, it is applicable to the laser light treatment using an endoscope. It is to be noted that the object to be treated with the present apparatus is not restricted.

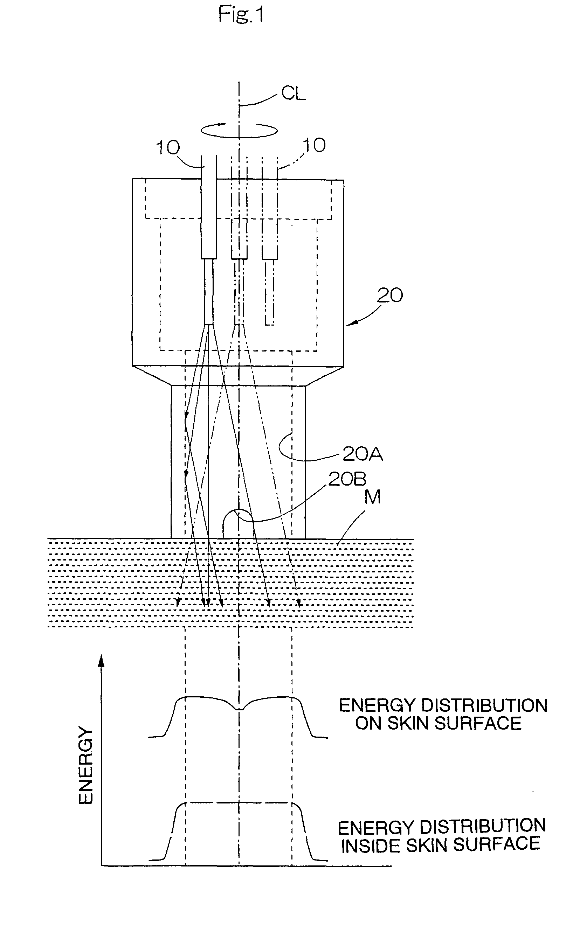

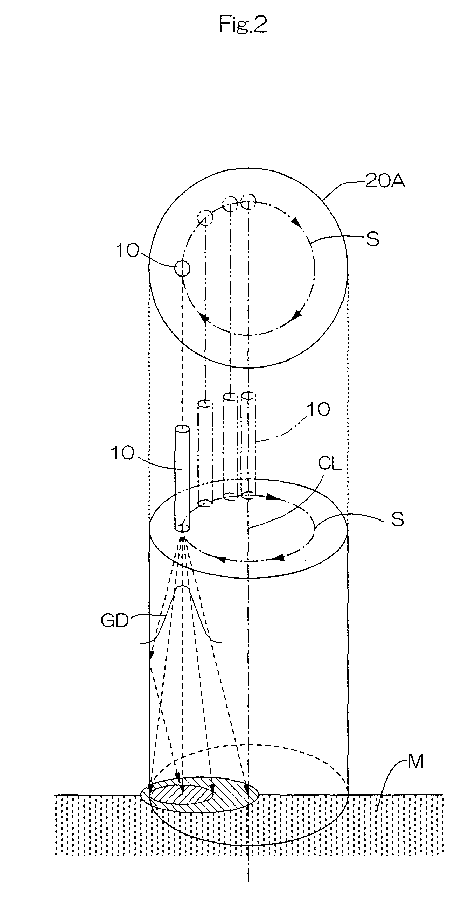

Now, basic concept of the present invention will be described with reference to Figs. 1 and 2. An optical fiber 10 is used as a laser light transmitting member of the present invention. Laser light from laser light generating source is incident upon the rear end of the optical fiber 10 and is transmitted therethrough and is emitted from the front end thereof and is incident upon the living tissue M, such as the skin. A clad is removed from the illustrated optical fiber 10 at the front end thereof so that its core is exposed. The optical fiber 10 is held by holding means such as handpiece.

Within the holding means, there is provided turning means including a motor for moving the tip end portion of the optical fiber 10 along a circular turning locus S substantially around a turning center axis CL. The tip end of the optical fiber 10 is turned along the circular turning locus S around the turning center axis CL at a high r.p.m., such as a few tens to a few ten thousands of r.p.m. Accordingly, the optical axis of the laser light also traces a circular locus in the cross section.

At each position, the laser light is emitted from the tip end of the optical fiber 10 in the course of high speed turning of the optical fiber along the turning locus S to exhibit Caussian distribution. The Gaussian distribution of the energy of the incident laser light GD in a longitudinal section is shown in Fig. 2.

The energy distribution of the laser light which is incident upon the tissue M is a resultant Caussian distribution of the laser light at each position. As a result, the energy distribution of the laser light which is incident upon the tissue M depends upon the diameter of the turning locus S. It exhibits the triangular shape having a gradual summit when the diameter is small. It exhibits the shape of somma when the diameter is large. In either case, the energy distribution of the laser light of the present invention which irradiates the skin surface exhibits a uniform energy distribution having a low peak height and which is relatively higher over a wider area.

Therefore, by appropriately selecting the relation among the diameter of the turning locus S, the distance between the tip end of the optical fiber 10 and the tissue, and the energy of the laser light which is emitted from the tip end of the optical fiber 10, the entire area of the object of the tissue M can be irradiated with the laser light so that the energy distribution over the tissue M is uniform. As a result, the tissue can be positively and uniformly irradiated with the laser light over the wider area thereof without giving any local damage to the tissue.

In the present invention, it is preferable to dispose a reflector cylinder 20 which reflects the laser light on the inner surface 20A in front of the tip end of the optical fiber 10 in an coaxial manner with respect to the turning center axis CL. The reflector cylinder 20 may be made of a plastic material such as acrylic resin and polycarbonate resin, inorganic material such as glass, and metal. In order that the surface of the tissue M is visible through the wall of the reflector cylinder 20, the cylinder 20 may be preferably transparent or semitransparent. From the view point of this, acrylic resin, polycarbonate resin, class, quartz, sapphire are preferable.

Disposition of the reflector cylinder 20 causes part of the laser light emitted from the tip end of the optical fiber 10 to be totally reflected on the inner surface 20A of the reflector cylinder 20 and part of it to be transmitted therethrough and the rest to be reflected and to be incident to the tissue M. Accordingly, since the laser light which is reflected on the inner surface 20A of the reflector cylinder 20 is additively incident upon the tissue M as shown in Figs. 1 and 2, the irradiation energy of the laser light on the tissue M exhibits substantially inverted U shape, so that the wider area of the object tissue M can be uniformly irradiated with laser light.

The laser light which has been incident upon the surface of the tissue M is scattered within the tissue M. In case in which a portion below the surface of the tissue M, for example, the blotch of the skin is to be evaporated, in order to irradiate a position which is about 1 to 3 mm deeper from the surface of the skin with laser light in a uniform manner, it has been found that the energy distribution of the laser light on the surface of the tissue M preferably be slightly lower around the turning center axis CL and higher at the periphery thereof.

In order to also obtain such an energy distribution of the laser light on the surface of the tissue M, it is effective to use the reflector cylinder 20, which enables the energy level of the irradiation of the object tissue M to increase at the periphery thereof.

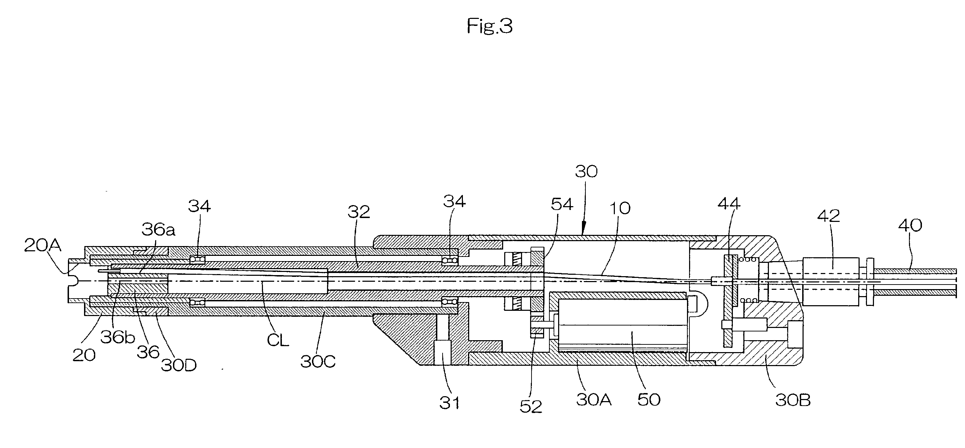

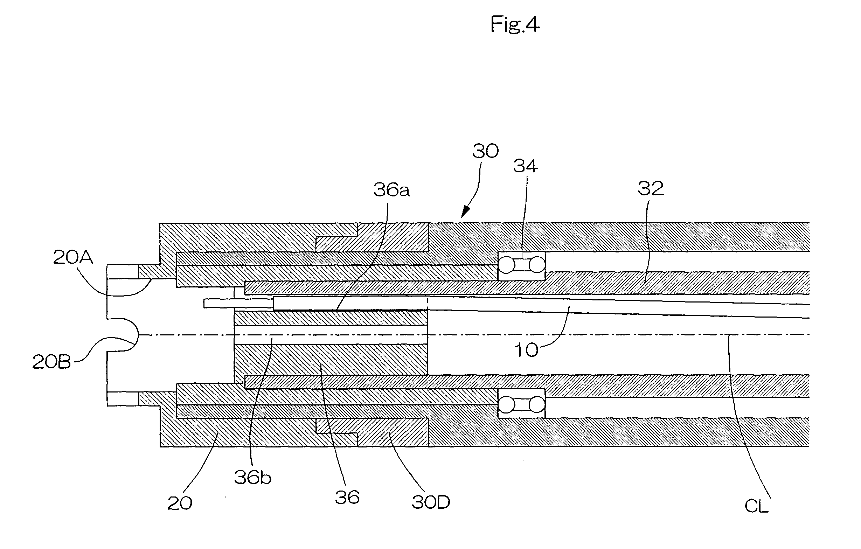

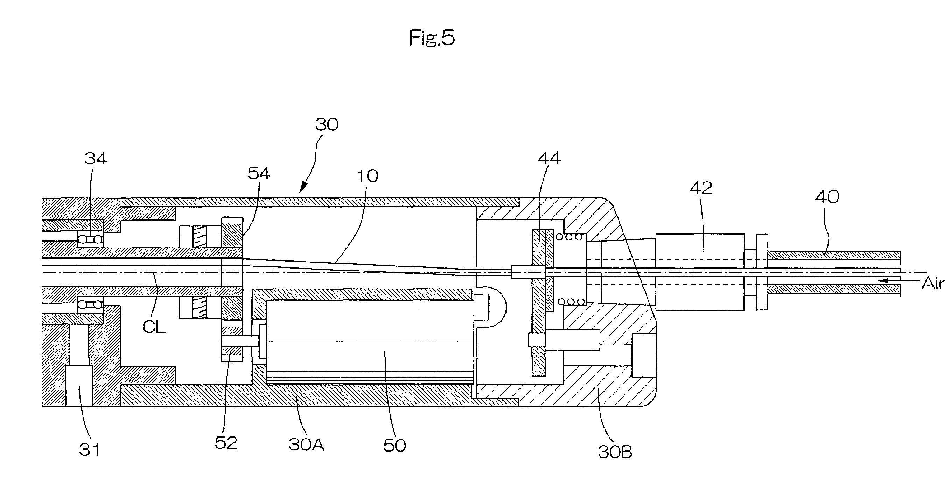

An exemplary configuration of the laser light irradiating apparatus is shown in Figs. 3 to 5, which is suitable for irradiating the tissue surface while it is gripped by a surgeon. This laser light irradiating apparatus comprises a casing 30 (handpiece) which serves as holding means and a hollow guide 32 which is disposed within the casing 30 in such a manner that it is rotatable around the turning center axis CL. The casing 30 has a removable cover 30B in the rear of a grip body 30A. A support cylinder 30C is integral with the grip body 30 at the front end thereof by means of a set screw 31. The reflector cylinder 20 is removably mounted on the front end of the support cylinder 30C via a coupler 30D.

The hollow tubular guide 32 is held in the support cylinder 30C by front and rear bearings 34 and 34 so that it is rotatable around the turning center axis CL. The optical fiber 10 is inserted into the casing 30 through the cover 30B of the casing 30, and further inserted inside the tubular guide 32. A holding member 36 is secured integrally within the tip end of the tubular guide 32, and the front end of the optical fiber 10 is held in a groove 36a which is formed on the outer periphery of the holding member 36.

As a result, the front end portion of the optical fiber 10 is held in a position offset from the turning center axis CL with the turning center axis CL due to the fact that the optical fiber is inserted with play inside the groove 36a on the outer periphery of the holding member 36.

A motor 50 which constitutes turning means is secured within the grip body 30A. A prime mover gear 52 is secured to the output shaft of the motor 50. The prime move gear 52 is meshed with a follower gear 54 which is integral with the rear portion of the tubular guide 32. Accordingly, the torque of the motor 50 is transmitted to the prime mover gear 52 and the follower gear 54, so that the tubular guide 32 is rotated around the turning center axis CL. It is preferable that the ratio of the number of the teeth of the gear (follower gear 54) on the side of the guide of the gear mechanism for transmitting the torque to the number of the teeth of the gear (prime mover gear 52) on the side of the motor is not a whole number, but a fraction. In the embodiment, the ratio is set to 1/32. Since the tissue is irradiated with laser light at different positions on the turning locus S in such an arrangement, the area of the tissue can be uniformly irradiated with laser light within a given period of time.

Since the front end portion of the optical fiber 10 is inserted with play inside the groove 36a formed on the outer periphery of the holding member 36 at this time, the optical fiber 10 itself does not follow the rotation of the tubular guide 32 and the holding member 36, nor is twisted therefore. The front end portion of the optical fiber 10 only traces a turning locus S.

The optical fiber 10 is optically coupled with the laser light supplying means. The laser light supplying means may include a laser light generator and pulsating means (both not shown). The laser light from the laser light supplying means is incident upon the rear end of the optical fiber 10 and is transmitted therethrough and is emitted from the front end thereof so that it is incident upon the tissue M of a living body, for example, the skin. The intermediate portion of the optical fiber 10 extends through a guide tube 40, a holder 42 which is secured to the cover 30B, to the inside of the cover 30B.

A space between the guide tube 40 and the optical fiber 10 serves as an air supplying passage. Through this passage and a passage formed within the holder 42 a cooling air is pumped into the casing 30 as a cooling medium by a compressor (not shown). The cooling air which has been pumped into the casing 30 is passed through the tubular guide 32 and a hollow 36b of the holding member 36, then enters into the reflecting cylinder 20 and is blown out from total 4 openings 20B which are formed at angularly 90 degrees spaced different positions on the wall of the front end.

The reflector cylinder 20 is removably mounted on the front end of the support cylinder 30C via the coupler 30D. The reflector cylinder 20 which is shown in Figs. 3 to 5 has a short inner surface 20A. It is preferable to provide a plurality of reflector cylinders having different length inner surfaces 20A. A reflector cylinder 20 having a longer inner surface 20A is shown in Fig. 1. By changing the manner of reflecting of the laser light on the inner surface 20A by selecting an appropriate reflecting cylinder among the plurality of reflecting cylinders depending upon the situation, the irradiating energy distribution of the laser light with respect to the tissue M can be changed. The energy of the laser light which is incident upon the tissue can also be changed even if the energy of the laser light emitted from the front end of the optical fiber 20 is made constant.

It is preferable to provide cooling medium pumping means for pumping a cooling medium into the reflector cylinder 20 to cool the surface of the tissue. Since the skin is irradiated with laser light without applying anesthesia, the skin which is irradiated with the laser light may be heated so that the patient can not endure it. In this case, the surface of the tissue M can be cooled by pumping a cooling medium into the reflector cylinder for mitigating the hot feeling of the patient. The cooling medium may include air, water, alcohol, carboxymethylcellulose. A liquid in which a high molecular gel is mixed with water or alcohol for adjusting its viscosity is effective. The volatile liquids have high cooling effect due to their vaporization heat. The cooling medium may be continuously or intermittently pumped into the reflecting cylinder. If the reflector cylinder 20 is formed on its wall with the opening 20B, the cooling medium would be pumped out from an opening 20B, so that the heat which is recovered by the cooling medium can be discharged to the outside.

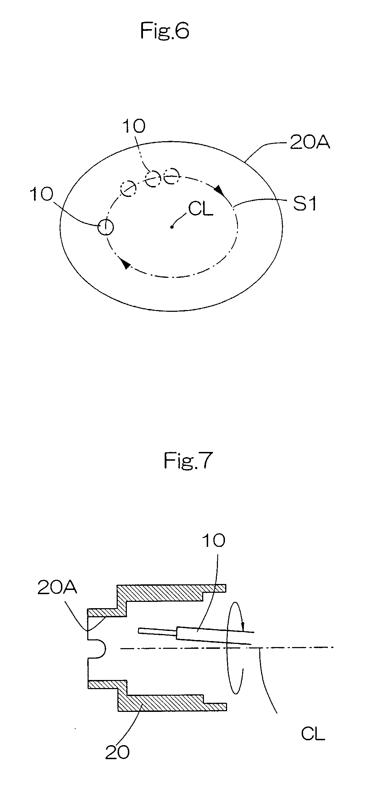

Although the optical fiber 20 traces the circular turning locus S in the foregoing embodiment, it may trace other shape such as elliptical turning locus S as shown in Fig. 6. Means for this purpose such as cam is readily conceivable for those skilled in the art. The optical axis of the optical fiber 10 may not be parallel to the turning center axis CL, but slightly inclined thereto.

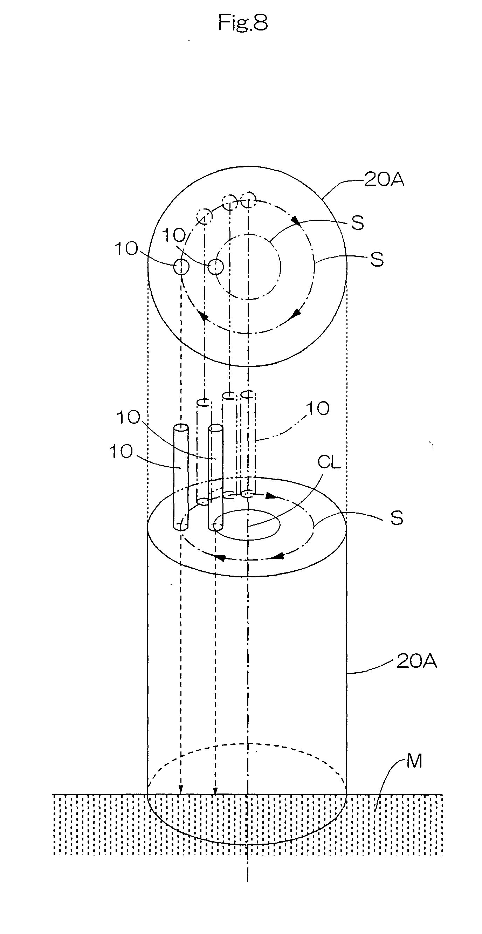

A plurality of the optical fiber 10 may be disposed. For example, they may be on the turning locus at different positions and may be disposed at different radius positions as shown in Fig. 8. If the irradiation area of the object is constant, the irradiation energy which is a multiple of the irradiation area per unit time can be provided and the irradiation area of the object can be widened.

The turning means may be disposed externally of the casing. The reflector cylinder may be omitted if necessary. The optical fiber may be replaced with glass or quartz rod as the laser light transmitting member.

The laser light having a wave length, from which the wave length not higher than 650 nm is eliminated may be preferably used, for example, the treatment of the blotch on the skin. For example, laser light having a length of 650 nm or more from krypton lamp, alexandrite (wave length 755 nm), in particular Nd:YAG laser (wave length 1064 nm) is preferable.

Continuous or pulsated laser light may be preferably used. The pulsated laser light is emitted at, for example, 5 to 10 pps. The output of the laser light is preferably 0.1 to 35 per one pulse.

## Claims

1. A laser light irradiating apparatus for irradiating the surface or inside of a living body tissue with laser light from laser light supplying means, comprising:
laser light transmitting member having a rear end to which the laser light from said laser light supplying means is incident and a front end from which the laser light is emitted;
holding means for holding said laser light transmitting member; and
turning means for moving a front end portion of the laser light transmitting member having at least said front end substantially along a turning locus around a turning center axis within said holding means.

2. A laser light irradiating apparatus as defined in Claim 1 in which a reflector cylinder for reflecting laser light on its inner surface is disposed in front of at least said front end of said laser light transmitting member coaxially with said turning center axis for reflecting part of the laser light emitted from said front end of said laser light transmitting member on said inner surface so that the reflected laser light is incident upon said tissue.

3. A laser light irradiating apparatus as defined in Claim 2 in which said reflector cylinder is transparent or semitransparent so that said tissue within said reflector cylinder is visible therethrough.

4. A laser light irradiating apparatus as defined in Claim 2 in which said reflector cylinder is removably mounted on said holding means and a plurality kinds of said reflector cylinders having different length are provided.

5. An laser light irradiating apparatus for irradiating the surface or inside of a living body tissue with laser light from laser light supplying means, comprising:
laser light transmitting member having a rear end to which the laser light from said laser light supplying means is incident and a front end from which the laser light is emitted;
holding means for holding said laser light transmitting member;
turning means for moving the front end portion of the laser light transmitting member having at least said front end substantially along a turning locus around a turning center axis within said holding means; and
a reflector cylinder for reflecting laser light on its inner surface which is mounted on said holding means coaxially with said turning center axis and is disposed in front of at least said front end of said laser light transmitting member for reflecting part of the laser light emitted from said front end of said laser light transmitting member on said inner surface so that the reflected light is incident upon said tissue.

6. A laser light irradiating apparatus as defined in Claim 5 and further including cooling medium pumping means for pumping a cooling medium which cools the surface of said tissue into the inside of said reflector cylinder.

7. A laser light irradiating apparatus as defined in Claim 5 in which said reflector cylinder has on its wall an opening, said cooling medium being continuously or intermittently pumped into said reflector cylinder by said cooling medium pumping means and being pumped out from said opening.

8. A laser light irradiating apparatus as defined in Claim 5 in which said cooling medium is adapted to be pumped into said reflector cylinder via the inside of said holding means.

9. A laser light irradiating apparatus as defined in Claim 5 in which said holding means comprises a casing and a guide which is disposed within said casing in such a manner that the guide is rotatable around said turning center axis, said laser light transmitting member being an optical fiber, said turning means being a motor which is disposed within said holding means, and in which at least a front end portion of said optical fiber is held by being inserted into said guide with play at a position offset from said turning center axis in such a manner that it is in substantially parallel with said turning center axis, said guide being disposed to be rotated by a torque from said motor.

10. A laser light irradiating apparatus as defined in Claim 9 in which the torque of said motor is transmitted to said guide via a gear mechanism for rotating the guide.

11. A laser light irradiating apparatus as defined in Claim 10 in which the ratio of the number of teeth of a gear on the side of said guide of said gear mechanism to that of the gear on the side of said motor is not a whole number, but a fraction.

12. A laser light irradiating apparatus as defined in Claim 5 in which said laser light transmitting member is held at different positions on the turning locus by a plurality of holding means.

13. A laser light irradiating apparatus as defined in Claim 5 in which a plurality of said laser light transmitting members are disposed, which are held at different radius positions from said turning center axis by said holding means.

14. A laser light irradiating apparatus as defined in Claim 5 in which said laser light transmitting member is held on the turning locus at different positions by a plurality of holding means.

15. A laser light irradiating apparatus as defined in Claim 5 in which said laser light supplying means supplies said laser light transmitting member with YAG pulse laser light for irradiating the skin of a living body with the laser light.
